# EUROPEAN PATENT SPECIFICATION

(11) **EP 1 482 879 B1**
(45) Date of publication and mention of the grant of the patent: **07.10.2009**
(21) Application number: 03716516.4
(22) Date of filing: 11.03.2003
(51) Int. Cl.: A61F 5/055

(54) **CERVICAL COLLAR HAVING ENHANCED LATERAL SUPPORT**
HALSKRAGEN MIT VERBESSERTER SEITLICHER STÜTZE
COLLET CERVICAL AVEC SUPPORT LATERAL AMELIORE

(30) Priority: 11.03.2002 US 363416 P
(43) Date of publication of application: 08.12.2004
(73) Proprietor: Össur hf, 110 Reykjavik (IS)
(72) Inventor: Calabrese, Salvatore, Philadelphia, Pennsylvania 19145 (US)
(74) Representative: W.P. Thompson & Co.
(86) International application number: PCT/US2003/007655
(87) International publication number: WO 2003/077793

(56) References cited:
- US-A- 3 042 027
- US-A- 3 756 226
- US-A- 4 782 824

## Description

### Field of the Invention

This invention relates generally to cervical collars.

### Background of the Invention

Head immobilization is necessary when a person has suffered trauma to his head, neck, and/or spine. Various cervical collars are generally known to provide such head immobilization. One such collar is a two-piece cervical collar fabricated from a soft, flexible, light-weight material, for example a non-injection molded plastic material such as polyethylene or polyurethane: see for example US-A-3756226. These cervical collars provide the necessary head immobilization needed for most neck and head injuries and also provide a increased degree of comfort due to their soft flexible construction. However, in some instances, enhanced lateral support may be necessary to provide an increased degree of head immobilization in the lateral direction and/or rotational support.

### Summary

The present invention provides a cervical collar comprising a front collar portion having a chin support portion and generally conforming to the neck, shoulders, and jawbone of a wearer. The front collar portion is fabricated from a soft flexible material and a lateral reinforcing support member disposed in the neck region below the jawbone of the wearer. The support member is fabricated from a substantially incompressible material and positioned on the collar such that, in use of the collar, an upper front corner of the support member is positioned to engage the wearer's mandible to limit rotation of the wearer's head.

The support member can be substantially flat. The upper front corner engaging the wearer's neck can be rounded. The support member can be attached to the front collar portion with one or more rivets. A plurality of the support members may be provided on each lateral side of the wearer's neck.

In one form of the invention, the support member is fabricated from a lightweight and rigid material for providing sufficient lateral support to the member's head.

The front collar portion may be fabricated from a foam material. A fabric fastener for connecting the front collar half to a rear collar half may be employed. The fabric may be attached to the front collar portion by a rivet attaching the support member to the front collar portion.

In one form of the invention, a plurality of support members may be provided to increase the lateral support. In addition, the support member may have a colour which is indicative of a material property of the support member.

### Brief Description of the Drawings

For the purpose of illustrating the invention, there is shown in the drawings an embodiment which is presently preferred; it being understood, however, that this invention is not limited to the precise arrangement and instrumentalities shown.
Fig. 1 is a front elevational view of a preferred embodiment of a cervical collar of the present invention.
Fig. 2 is a side view of the cervical collar of Fig. 1;
Fig. 3 is a back elevational view of the cervical collar of Fig. 1 with a portion of the fabric fastener folded back;
Fig. 4 is a view corresponding to that of Fig. 2 but with a fastener folded back and
Fig. 5 is a sectional view taken on the line V-V on Fig. 4.

### Detailed Description of the Preferred Embodiment

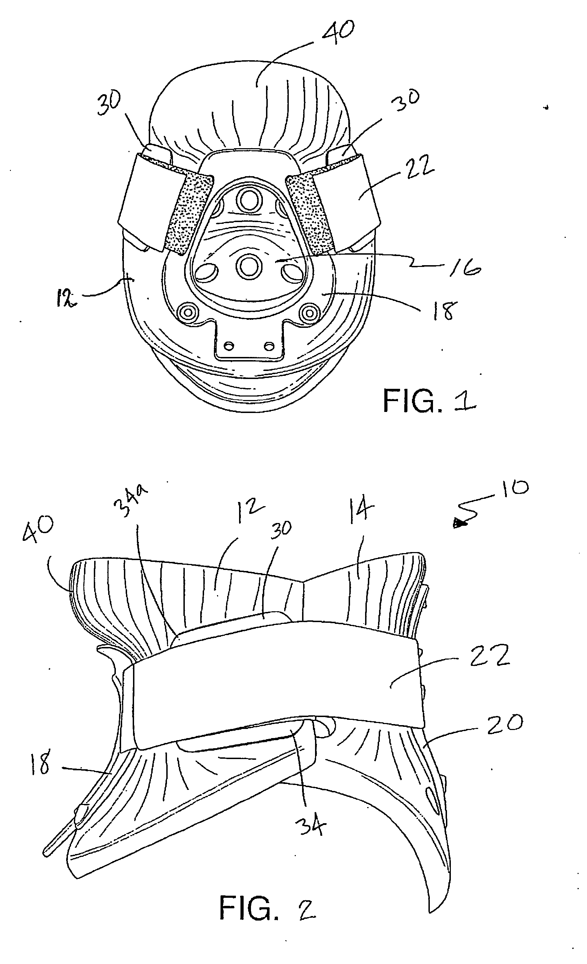

Referring to the drawings, wherein like numerals indicate like elements, there is shown in Figs. 1 - 5 a cervical collar 10 having a front half collar portion 12 and a separate rear half portion 14. The cervical collar of the present invention is described generally in U.S. Patent Nos. 3,756,226 and 4,677,962 to Calabrese.

The front half collar portion 12 is for application to the front of a wearer's neck region and generally comprises a U-shaped body preferably fabricated from a soft, flexible, light-weight material which is preferably non-toxic, hypo-allergenic, latex-free, water-resist and buoyant in water, for example a non-injection molded closed cell polymeric plastic material such as polyethylene or polyurethane. The front collar portion 12 has a chin support portion 40 for supporting the wearer's chin and is generally shaped to conform to the front of the wearer's chin, mandible, neck, shoulders, and upper chest.

Similarly, the rear half collar portion 14 is for application to the rear of a wearer's neck region and is fabricated from a similar material as that of front half portion 12. The rear half collar portion is generally shaped to conform to the rear of the wearer's chin, mandible, neck, shoulders, and back/spine.

The front collar half 12 can be provided with an opening 16 below the chin support 40 for providing access to the tracheal region of the person's neck and for otherwise providing ventilation to the neck region.

Referring back to Fig. 1, the front half collar portion 12 is also provided with a rigid reinforcing support member 18 fabricated from an incompressible plastic material which is stiff and generally inflexible. Typically, the support member 18 can also have a tracheal opening similar to opening 16.

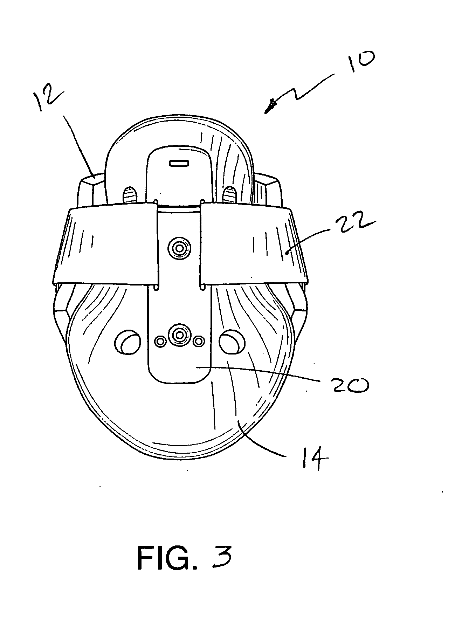

Similarly, as best shown in Fig. 3, the rear half portion 14 is provided with a similar reinforcing support member 20 which is fabricated from the same material as the support member 18. The front and rear support members 18 and 20 function to limit or decrease the flexion and extension of a wearer's head in the front and rear direction respectively. The support members 18 and 20 can also be provided with additional connection elements, such as openings and/or slots, as is well known in the art. In addition, the support members 18 and 20 can also be provided with slots, of the like, for attachment of fabric fasteners for joining the front and rear collar halves around the neck of a wearer.

In addition, a hook and loop fabric fastener 22 is provided for connecting the front and rear halves of the collar together. One strip of fabric fastener strip is connected to the rear collar half, typically to the rear support member 20. Similarly, a second fabric fastener strips are connected to the front collar half 12, typically to the front support member 18. The free ends of the fastener strips are attached to secure the collar halves together.

The cervical collar 10 is provided with lateral reinforcement support members or plates 30 for reducing the range of lateral and rotational motion of a wearer's head. The lateral reinforcement support members 30 are located on both sides of the cervical collar 10 in the wearer's neck region and they may be attached to the collar 10 by any suitable means, such as by rivets 32, adhesive, sonic welding, molding in place, or the like. In addition, the lateral reinforcing members 30 can be fabricated by any lightweight and rigid material to achieve the desired lateral and rotational support.

Moreover, the fabric fastener strips can be fastened to the collar halves in the same manner as the lateral supports 30. In the embodiment shown, rivets 32 fasten the fasten strips and the support members 30 to the front collar half portion 12.

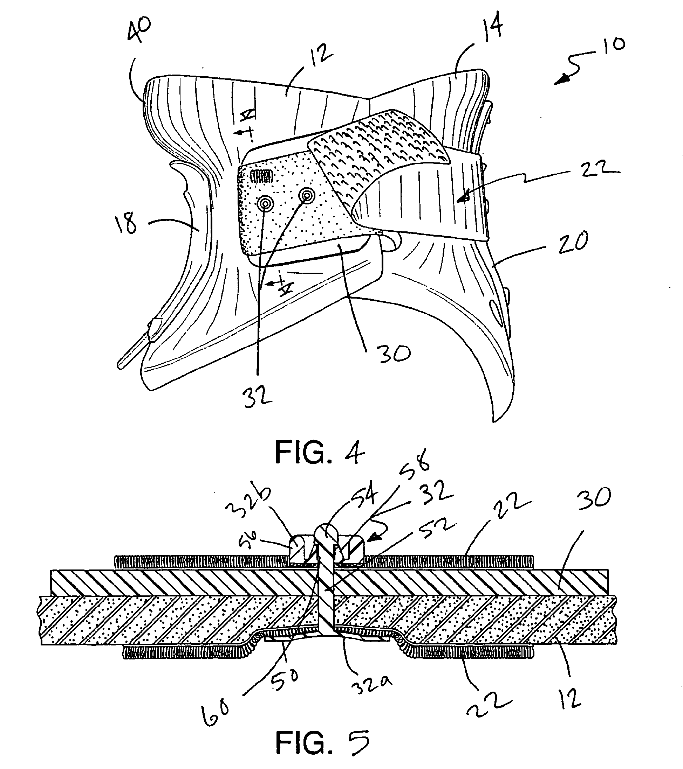

As best seen in Fig. 5, one preferred rivet design is illustrated. The rivet 32 comprise a male portion 32a and a female portion 32b. The male portion includes an enlarged end portion and an upstanding male member 52 having an enlarged rounded head 54. The female portion 32b has an upstanding peripheral wall 56, a medial upstanding circular wall 58, and a central opening 60 inside the medial wall 58. To engage the male and female rivet portions, the enlarged head 54 of the male member 52 is passed through the medial opening 60 of the female portion and the bottom radial surface enlarged head is trapped on the medial wall 58 to prevent removal.

Although the supports 30 are shown as flat plates with rounded corners 34, it is contemplated that the lateral reinforcement support members 30 can be of various thicknesses, and rigidities to provide the desired degree of lateral support. For example, the lateral reinforcement support members 30 can be fabricated as a thin-walled structure having additional structural reinforcing supports or ribs (not shown). It is also contemplated that the lateral reinforcing support members 30 can be fabricated from any lightweight and rigid material that can achieve the desired support properties described herein, such as polypropylene or the like.

It is also contemplated that the lateral reinforcement members 30 can be offered in various sizes and rigidities so that the fitter, user, or physical therapist can vary the supports 30 by changing the number of supports used or the rigidity and/or size of the support(s) used to vary the amount of lateral and rotational support provided thereby.

It should be understood that the lateral reinforcement members 30 primarily serve to decrease the lateral flexion and rotation of the wearer's head over and above the support already provided by the foam collar 10. This lateral support can be used to limit the range of lateral and rotational motion or to provide resistance against lateral and rotational motion of the head so as to provide the necessary resistance to strengthen the neck muscles of the wearer employing the cervical collar 10 in a physical therapy or exercise regime.

The lateral reinforcing members 30 are located on the cervical collar 10 in the neck region of the wearer and below the mandible bone of the wearer such that the upper front corner 34a engages the mandible of the wearer to limit the rotation of the wearer's head. As such, it is contemplated that the lateral supports 30 are positioned parallel to the wearer's jawbone.

It is also contemplated that the lateral reinforcement support members 30 can be fabricated together with the front support 18 to facilitate fabrication, manufacture, and assembly of the collar. Or, alternatively, the lateral support members 30 can be fabricated separately to facilitate removal or replacement thereof as described above.

It is also contemplated that the lateral reinforcement supports 30 can be manufactured in a wide range of colours or can be manufactured using different colour schemes to compliment or enhance the colour of the cervical collar body itself.

The present invention may be embodied in other specific forms about departing from the essential attributes thereof and, accordingly, reference should be made to the appended claims, rather than to the forgoing specification, as indicating the scope of the invention.

## Claims

1. A cervical collar (10) comprising a front collar portion (12) having a chin support portion (40) and generally conforming to the neck, shoulders, and mandible of a wearer, the front collar portion (12) being fabricated from a soft flexible material, **characterised by** a lateral reinforcing support member (30) disposed in a neck region of the collar to lie in use of the collar below the mandible bone of the wearer, the support member being fabricated from a substantially incompressible material and being positioned on the collar such that, in use of the collar, an upper front corner (34a) of the support member is positioned to engage the mandible of the wearer to limit rotation of the wearer's head.

2. A cervical collar according to claim 1, wherein the support member is positioned on the collar such that, in use of the collar, the support member lies substantially parallel to the mandible of the wearer.

3. A cervical collar according to claim 1 or 2, wherein the support member (30) has a generally rectangular shape forming a plate.

4. A cervical collar according to any preceding claim, wherein the support plate (30) is substantially flat.

5. A cervical collar according to any preceding claim, wherein the said upper front corner support plate (30) has at least one rounded corner (34).

6. A cervical collar according to any preceding claim, wherein the support member (30) is fabricated from a lightweight and rigid material for providing sufficient lateral support to the wearer's head.

7. A cervical collar according to any preceding claim, wherein a plurality of said lateral reinforcing support members (30) are provided.

8. A cervical collar according to claim 7, wherein at least one support member is provided at each lateral side of the collar.

9. A cervical collar according to any preceding claim, wherein the or each support member (30) has a colour which is indicative of a material property of the support member.

10. A cervical collar according to any preceding claim, wherein the front collar portion (12) is fabricated from a foam material.

11. A cervical collar according to any preceding claim, wherein the or each support member (30) is fabricated separately from the remainder of the collar.

12. A cervical collar according to any of claims 1 to 10, wherein the or each support member (30) is attached to the front collar portion (12).

13. A cervical collar according to claim 12, wherein the or each support member (30) is attached to the front collar portion (12) by at least one rivet (32).

14. A cervical collar (10) according to claim 13, having a fabric fastener for connecting the front collar half (12) to a rear collar half (14), and wherein the fabric fastener is attached to the front collar portion (12) by a said rivet (32).

15. A method of fitting a cervical collar (10) comprising the steps of: providing a cervical collar (10) comprising a front collar portion (12) having a chin support portion (40) and generally conforming to the neck, shoulders, and mandible of a wearer, the front collar portion (12) being fabricated from a soft flexible material; fitting a lateral reinforcing support member (30) fabricated from a substantially incompressible material to the front collar portion (12) in the neck region below the mandible bone of the wearer, an upper front corner (34a) of the support member being positioned to engage the mandible of the wearer to limit rotation of the wearer's head; testing the lateral support provided by the lateral support member; and fitting at least one additional lateral support member to the front portion (12) depending upon the results of the test.

## Patentansprüche

1. Halskragen (10), aufweisend einen vorderen Kragenabschnitt (12), der einen Kinnstützabschnitt (40) besitzt und sich allgemein an den Hals, die Schultern und den Unterkiefer eines Trägers anpasst, wobei der vordere Kragenabschnitt (12) aus einem weichen, flexiblen Material hergestellt ist, **gekennzeichnet durch** ein seitliches, verstärkendes Stützteil (30), das in einer Halsregion des Kragens angeordnet ist, um bei Benutzung des Kragens unterhalb des Unterkieferknochens des Trägers zu liegen, das aus einem im Wesentlichen inkompressiblen Material hergestellt ist und an dem Kragen so positioniert ist, dass, bei Benutzung des Kragens, eine obere vordere Ecke (34a) des Stützteils positioniert ist, den Unterkiefer des Trägers zu fassen, um eine Drehung des Kopfes des Trägers zu begrenzen.

2. Halskragen nach Anspruch 1, wobei das Stützteil an dem Kragen so positioniert ist, dass, bei Benutzung des Kragens, das Stützteil im Wesentlichen parallel zu dem Unterkiefer des Trägers liegt.

3. Halskragen nach Anspruch 1 oder 2, wobei das Stützteil (30) eine im Wesentlichen rechtwinklige Form besitzt, die eine Platte bildet.

4. Halskragen nach einem vorhergehenden Anspruch, wobei die Stützplatte (30) im Wesentlichen flach ist.

5. Halskragen nach einem vorhergehenden Anspruch, wobei die die obere vordere Ecke aufweisende Stützplatte (30) zumindest eine abgerundete Ecke (34) besitzt.

6. Halskragen nach einem vorhergehenden Anspruch, wobei das Stützteil (30) aus einem leichten und starrem Material hergestellt ist, um ausreichend seitliche Stützung für den Kopf des Trägers zu bieten.

7. Halskragen nach einem vorhergehenden Anspruch, wobei eine Mehrzahl von seitlichen, verstärkenden Stützteilen (30) vorgesehen ist.

8. Halskragen nach Anspruch 7, wobei zumindest ein Stützteil an jeder seitlichen Seite des Kragens vorgesehen ist.

9. Halskragen nach einem vorhergehenden Anspruch, wobei das oder jedes Stützteil (30) eine Farbe besitzt, die auf eine Materialeigenschaft des Stützteils hinweist.

10. Halskragen nach einem vorhergehenden Anspruch, wobei der vordere Kragenabschnitt (12) aus einem Schaumstoff hergestellt ist.

11. Halskragen nach einem vorhergehenden Anspruch, wobei das oder jedes Stützteil (30) getrennt von dem Rest des Kragens hergestellt ist.

12. Halskragen nach einem der Ansprüche 1 bis 10, wobei das oder jedes Stützteil (30) an dem vorderen Kragenabschnitt (12) befestigt ist.

13. Halskragen nach Anspruch 12, wobei das oder jedes Stützteil (30) an dem vorderen Kragenabschnitt (12) durch mindestens einen Niet (32) befestigt ist.

14. Halskragen (10) nach Anspruch 13, mit einem Stoffverschluss zum Verbinden der vorderen Kragenhälfte (12) mit einer hinteren Kragenhälfte (14), und wobei der Stoffverschluss an dem vorderen Kragenabschnitt (12) durch einen besagten Niet (32) befestigt ist.

15. Verfahren zum Anlegen eines Halskragens (10), aufweisend die Schritte: Zurverfügungstellen eines Halskragens (10), aufweisend einen vorderen Kragenabschnitt (12), der einen Kinnstützabschnitt (40) besitzt und sich allgemein an den Hals, die Schultern und den Unterkiefer eines Trägers anpasst, wobei der vordere Kragenabschnitt (12) aus einem weichen, flexiblen Material hergestellt ist; Anbringen eines seitlichen, verstärkenden Stützsteils (30), das aus einem im Wesentlichen inkompressiblen Material hergestellt ist, an dem vorderen Kragenabschnitt (12) in der Halsregion unterhalb des Unterkieferknochens des Trägers, wobei eine obere vordere Ecke (34a) des Stützteils positioniert ist, den Unterkiefer des Trägers zu fassen, um eine Drehung des Kopfes des Trägers zu begrenzen; Testen der durch das seitliche Stützteil gegebenen seitlichen Stützung; und Anbringen wenigstens eines zusätzlichen seitlichen Stützteils an dem vorderen Abschnitt (12) in Abhängigkeit von den Ergebnissen des Tests.

## Revendications

1. Collier cervical (10) comprenant une partie de collier avant (12) présentant une partie de support de menton (40) et se conformant sensiblement au cou, aux épaules, et à la mandibule d'un porteur, la partie de collier avant (12) étant fabriquée en un matériau flexible souple, **caractérisé par** un élément de support de renforcement latéral (30) disposé dans une zone de cou du collier afin de se trouver, au cours de l'utilisation du collier, au-dessous de l'os de mandibule du porteur, l'élément de support étant fabriqué en un matériau sensiblement incompressible et étant positionné sur le collier de telle sorte que, au cours de l'utilisation du collier, le coin supérieur avant (34a) de l'élément de support est positionné de manière à se coupler à la mandibule du porteur afin de limiter la rotation de la tête du porteur.

2. Collier cervical selon la revendication 1, dans lequel l'élément de support est positionné sur le collier de telle sorte que, au cours de l'utilisation du collier, l'élément de support est agencé sensiblement parallèlement à la mandibule du porteur.

3. Collier cervical selon la revendication 1 ou 2, dans lequel l'élément de support (30) présente une forme sensiblement rectangulaire formant une plaque.

4. Collier cervical selon l'une quelconque des revendications précédentes, dans lequel la plaque de support (30) est sensiblement plate.

5. Collier cervical selon l'une quelconque des revendications précédentes, dans lequel ladite plaque de support de coin avant supérieur (30) comporte au moins un coin arrondi (34).

6. Collier cervical selon l'une quelconque des revendications précédentes, dans lequel l'élément de support (30) est fabriqué en un matériau léger et rigide afin d'assurer un support latéral suffisant pour la tête du porteur.

7. Collier cervical selon l'une quelconque des revendications précédentes, dans lequel il est agencé une pluralité desdits éléments de support de renforcement latéral (30).

8. Collier cervical selon la revendication 7, dans lequel au moins un élément de support est agencé au niveau de chaque face latérale du collier.

9. Collier cervical selon l'une quelconque des revendications précédentes, dans lequel le ou chaque élément de support (30) présente une couleur qui est représentative d'une propriété de matériau de l'élément de support.

10. Collier cervical selon l'une quelconque des revendications précédentes, dans lequel la partie de collier avant (12) est fabriquée en un matériau sous forme de mousse.

11. Collier cervical selon l'une quelconque des revendications précédentes, dans lequel le ou chaque élément de support (30) est fabriqué séparément par rapport au reste du collier.

12. Collier cervical selon l'une quelconque des revendications 1 à 10, dans lequel le ou chaque élément de support (30) est fixé sur la partie de collier avant (12).

13. Collier cervical selon la revendication 12, dans lequel le ou chaque élément de support (30) est fixé sur la partie de collier avant (12) par au moins un rivet (32).

14. Collier cervical (10) selon la revendication 13, comportant un élément de fixation en tissu afin de relier le demi-collier avant (12) au demi-collier arrière (14), et dans lequel l'élément de fixation en tissu est fixé sur la partie de collier avant (12) par un desdits rivets (32).

15. Procédé d'assemblage d'un collier cervical (10)
comprenant les étapes de : préparation d'un collier cervical (10) comprenant une partie de collier avant (12) présentant une partie de support de menton (40) et se conformant sensiblement au cou, aux épaules, et à la mandibule d'un porteur, la partie de collier avant (12) étant fabriquée en un matériau flexible souple ; assemblage d'un élément de support de renforcement latéral (30) fabriqué en un matériau sensiblement incompressible sur la partie de collier avant (12) dans la zone de cou au-dessous de l'os de mandibule du porteur, un coin supérieur avant (34a) de l'élément de support étant positionné de manière à se coupler à la mandibule du porteur afin de limiter la rotation de la tête du porteur ; contrôle du support latéral assuré par l'élément de support latéral ; et assemblage d'au moins un élément de support latéral supplémentaire sur la partie avant (12) en fonction du résultat du contrôle.
